# EUROPEAN PATENT SPECIFICATION

(11) **EP 4 635 453 B1**
(45) Date of publication and mention of the grant of the patent: **09.12.2026**
(21) Application number: 25169784.3
(22) Date of filing: 10.04.2025
(51) Int. Cl.: A61F 2/58, B25J 15/00

(54) **A PROSTHETIC HAND**
PROTHETISCHE HAND
PROTHÈSE DE MAIN

(30) Priority: 19.04.2024 GB 202405521
(43) Date of publication of application: 22.10.2025
(73) Proprietor: Open Bionics Ltd, Bristol BS1 2NB (GB)
(72) Inventor: WOOD, Steve, Bristol, BS1 2NB (GB); MEECH, James, Bristol, BS1 2NB (GB)
(74) Representative: Haseltine Lake Kempner LLP

(56) References cited:
- EP-B1- 2 890 333
- WO-A1-99/21517
- US-A1- 2009 326 677
- US-A1- 2010 198 362
- M.G. CATALANO ET AL: "Adaptive synergies for the design and control of the Pisa/IIT SoftHand", THE INTERNATIONAL JOURNAL OF ROBOTICS RESEARCH, vol. 33, no. 5, 1 April 2014 (2014-04-01), pages 768 - 782, XP055697508, DOI: 10.1177/0278364913518998

## Description

### FIELD OF THE INVENTION

The present disclosure relates to a prosthetic hand and particularly, although not exclusively, relates to a prosthetic hand with a battery housing at the wrist joint.

### BACKGROUND OF THE INVENTION

Controllers, drive mechanisms, and/or any other electrical components that may be present in a prosthetic hand may be powered by a battery. However, due to the size and shape of the batteries typically used, it can be difficult to ergonomically integrate the battery into the prosthetic hand. Indeed, batteries are often too large or too awkwardly shaped to be disposed in the prosthetic hand itself. Furthermore, integrating the battery in the prosthetic hand could limit a range of motion of the prosthetic hand. For these reasons, the battery is often disposed remote to the prosthetic hand, e.g. on a user's arm.

Electrical cables or wires may couple the battery on the user's arm to the electrical components in the prosthetic hand. For example, electrical cables may run from the battery, down the user's arm, and into the prosthetic hand. These electrical cables may suffer from an increased likelihood of becoming damaged or worn. The cables may become twisted, such as when the prosthetic hand is rotated relative to the arm. The prosthetic hand may therefore begin to operate at a substandard performance or break in a relatively short period of time. It is therefore desirable to improve a life expectancy of a prosthetic hand, and to reduce or eliminate a likelihood of the prosthetic hand needing to be replaced due to components (such as electrical cables) being damaged or worn.

In addition, running electrical cables or wires from a battery disposed on the user's arm and into the prosthetic hand may result in a design that permits fluids, such as water, to flow into the prosthetic hand. This may damage electronic components in the prosthetic hand, and accordingly, many prosthetic hands cannot be used in water above the wrist. It is therefore also desirable to provide a prosthetic hand with an improved robustness to water ingress.

Disposing a battery cell on a user's arm may also reduce an ability to modularise the prosthetic hand. For example, if there is a malfunction in the prosthetic hand, such as in a drive mechanism of the prosthetic hand, it may be required to remove internal electrical cabling, replace the faulty component, and then rethread the cables and reposition the battery. This may be distressing for a user, who may have to wait for a prolonged period of time before regaining use of their prosthetic hand. It may also be time-inefficient and expensive. Integrating a battery on a user's arm can therefore also add complexity and manufacturing time for a prosthetist. It is therefore also desirable to provide a prosthetic hand having an improved modular design with components that can be readily interchanged and replaced, for example, by the supplier of the prosthetic hand, the end user, or during assembly. Document US 2010/198362 A1 describes a prosthetic hand with a battery.

### SUMMARY OF THE INVENTION

According to a specific aspect, there is provided a prosthetic hand comprising:
a wrist attachment part configured for connecting to a prosthetic arm;
a palm part, the palm part and wrist attachment part being rotatably coupled together to at least partially form a wrist joint with a lateral axis of rotation; and
a battery housing, wherein the battery housing is positioned such that the axis of rotation of the wrist joint passes through the battery housing.

The battery housing may form a shaft configured to rotatably couple the wrist attachment portion and the palm part.

A longitudinal axis of the battery housing may be substantially parallel and coincidental to the lateral axis of rotation of the wrist joint.

A cavity may at least partially extend through the palm part. The battery housing may be disposed in the cavity. The cavity may alternatively be a void or through-hole.

The wrist attachment part may comprise a first casing part and a second casing part. The first casing part may at least partially surround a first portion of the palm part. The second casing part may at least partially surround a second portion of the palm part. The first casing part and the second casing part of the wrist attachment part may be decouplable from one another.

One of the palm part or the wrist attachment part may comprise at least one recess. The other of the palm part or the wrist attachment part may comprise at least one corresponding shaft. The at least one corresponding shaft may be configured to extend into the at least one recess to thereby rotatably couple the palm part to the wrist attachment part.

The wrist attachment part may comprise a first lateral side opening and a second lateral side opening. The palm part may comprise a through hole. The first lateral side opening of the wrist attachment part may be aligned to one end of the through-hole. The second lateral side opening of the wrist attachment part may be aligned to the other end of the through-hole. The battery housing may extend from the first lateral side opening of the wrist attachment part, through the through-hole of the palm part, and into the second lateral side opening of the wrist attachment part, to thereby rotatably couple the palm part to the wrist attachment part.

The palm part may be coupled to a bridging part. The bridging part may be disposed proximate to the wrist attachment part. The bridging part may be at least partially surrounded by the wrist attachment part. The through-hole may be formed between the bridging part and the palm part.

The bridging part and the palm part may be decouplable from one another.

The wrist attachment part may comprise a first casing part and a second casing part. The first casing part may at least partially surround a first portion of the bridging part. The second casing part may at least partially surround a second portion of the bridging part.

The first casing part and the second casing part of the wrist attachment part may be decouplable from one another.

The first casing part may comprise the first lateral side opening. The second casing part may comprise the second lateral side opening.

The battery housing may comprise a first frustoconical end and/or a second frustoconical end. The first lateral side opening of the wrist attachment part may be disposed around a periphery of a smaller diameter surface of the first frustoconical end. The second lateral side opening of the wrist attachment part may be disposed around a periphery of a smaller diameter surface of the second frustoconical end.

The battery housing may comprise a first part and a second part. The first part of the battery housing may be separable from the second part of the battery housing. The first part of the battery housing may comprise the first frustoconical end and a joining portion. The second part of the battery housing may comprise the second frustoconical end and a joining portion. A leading surface of the joining portion of the first part of the battery housing may abut or contact a leading surface of the joining portion of the second part of the battery housing to define an interface surface. The interface surface may be configured to surround a battery.

The interface surface may at least partially surround a geometric centre of the battery housing. The first part of the battery housing may be configured to house a first half of a battery and the second part of the battery housing is configured to house a second half of the battery.

The battery housing may comprise a first part and a second part. The first part may be separable from the second part. The first part may be configured to house a first portion of a battery. The second part may be configured to house a second portion of the battery.

The palm part may be couplable to at least one prosthetic digit.

The at least one prosthetic digit may comprise a first digit, a second digit, a third digit, a fourth digit, and a fifth digit.

The first digit may be a prosthetic thumb, the second digit may be a prosthetic index finger, the third digit may be a prosthetic middle finger, the fourth digit may be a prosthetic ring finger, and the fifth digit may be a prosthetic little finger.

The prosthetic hand may comprise at least one driving system configured to control a movement of the at least one prosthetic digit. The at least one driving system may be configured to be electrically coupled to a battery housed in the battery housing.

The at least one controller may be configured to control the at least one driving system based on at least one received signal. The at least one received signal may be configured to be wirelessly communicated to the controller.

The at least one received signal may be at least one signal generated by at least one sensor. The at least one sensor may be an electromyography sensor. The at least one sensor may be configured to be disposed adjacent to a residual limb of a user. Wireless communication may be by any wireless communication protocol, such as by Bluetooth^{®}.

The wrist attachment part may comprise a mating part. The mating part may be configured to enable the wrist attachment part to be connected to a United States Manufacturing Company (USMC) style connector. Additionally or alternatively, the mating part may be configured to enable the wrist attachment part to be connected to any other connector, and/or any other quick release/modular prosthetic socket.

The battery housing may be configured to house a rechargeable battery. One of the palm part or the wrist attachment part may comprise a port. The port may be configured to permit an external power source to be electrically coupled to the rechargeable battery. The port may be a USB-C port.

The battery housing may be configured to house an 18650 battery cell.

The battery housing may comprise at least one enlargement part. The enlargement part may be configured to increase an internal volume of the battery housing.

An external surface of the battery housing may comprise at least one groove. The at least one groove may be configured to locate a sealing ring.

The battery housing may comprise a first groove and a second groove. Each groove may be configured to locate a sealing ring. The first groove may be disposed between the at least one enlargement part and a first end of the battery housing. The second groove may be disposed between the at least one enlargement part and a second end of the battery housing.

The palm part may be rotatable relative to the wrist attachment part through a plurality of discrete positions.

One of the palm part or the wrist attachment part may comprise at least notch. The other of the palm part or the wrist attachment part may comprise at least one protrusion. The at least one protrusion may be configured to extend into or retract from the at least one notch due to the palm part being rotated relative to the wrist attachment part.

According to another specific aspect, there is provided a prosthetic foot comprising:
an ankle attachment part configured for connecting to an ankle connector;
a foot part, the foot part and ankle attachment part being rotatably coupled together to at least partially form an ankle joint with a lateral axis of rotation; and
a battery housing, wherein the battery housing is positioned such that the axis of rotation of the ankle joint passes through the battery housing.

These and other aspects will be apparent from and elucidated with reference to the embodiment(s) described hereinafter. Also, features described with respect to one aspect or embodiment may also be applied to other aspects or embodiments.

### BRIEF DESCRIPTION OF THE DRAWINGS

Exemplary embodiments will now be described, by way of example only, with reference to the following drawings, in which:
Figure 1 is a perspective view of a prosthetic hand according to an example of the present disclosure;
Figure 2 is a perspective view of a prosthetic hand comprising digits according to an example of the present disclosure;
Figure 3 is a perspective view of a bridging part and a palm part of a prosthetic hand according to an example of the present disclosure;
Figure 4 is a perspective view of the bridging part, palm part, and a battery housing of the prosthetic hand according to an example of the present disclosure;
Figure 5 is a front view of the battery housing of the prosthetic hand according to an example of the present disclosure; and
Figure 6 is a front view of a first part of the battery housing and a battery of the prosthetic hand according to an example of the present disclosure.

### DETAILED DESCRIPTION OF EMBODIMENTS

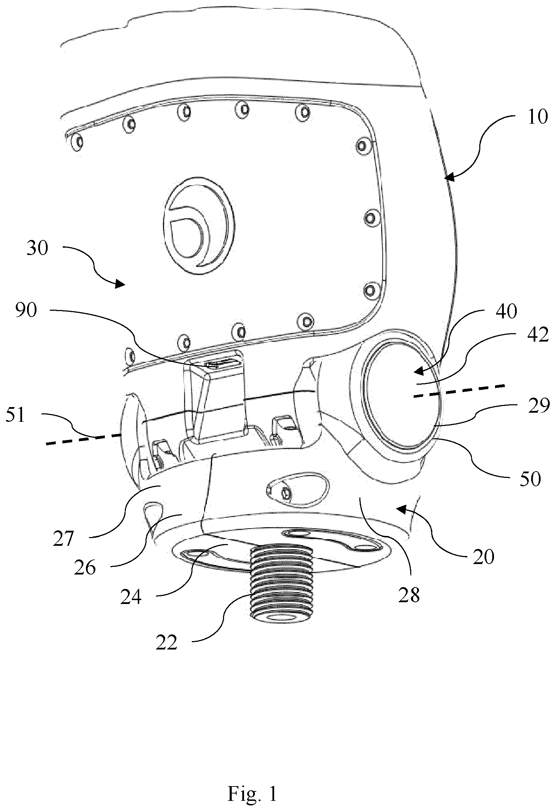

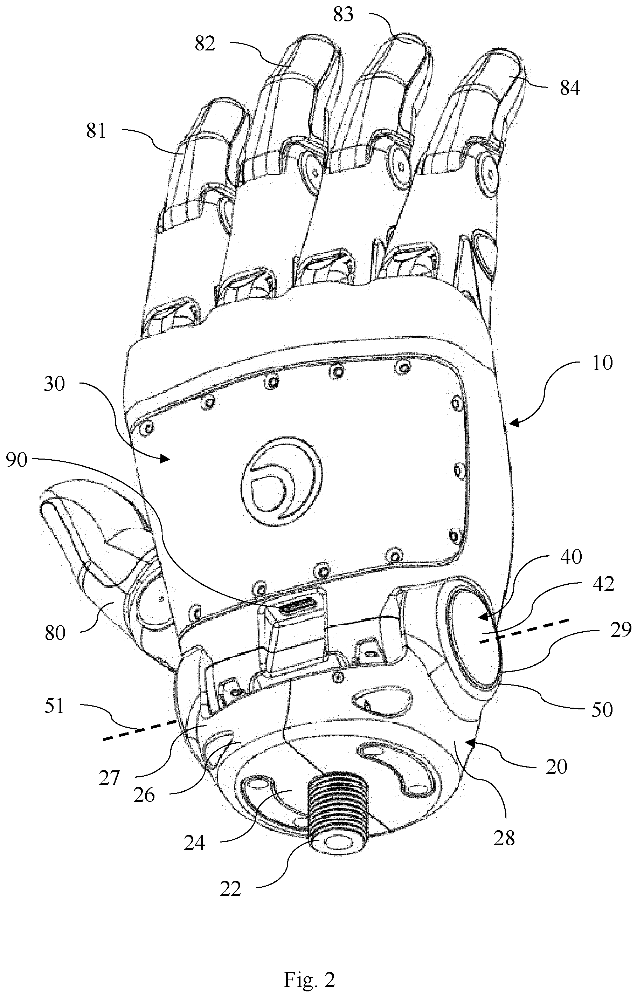

With reference to Figures 1 and 2, a prosthetic hand 10 according to an example of the present disclosure comprises a wrist attachment part 20, a palm part 30, and a battery housing 40.

The wrist attachment part 20 is configured to be connected to a prosthetic arm. The wrist attachment part 20 may alternatively be termed an arm attachment part. The wrist attachment part 20 may comprise a mating part 22. As shown in Figures 1 and 2, the mating part 22 may be a male mating part. The mating part 22 may alternatively be a female mating part or any other suitable mating part. The mating part 22 may be configured to be connected or coupled to a corresponding mating part of the prosthetic arm. Although not shown, the corresponding mating part of the prosthetic arm may be any type of connector, such as a United States Manufacturing Company (USMC) style connector. The mating part 22 may comprise a thread. The thread may permit a plurality of different components to be fastened or secured adjacent to the wrist attachment part 20. For example, a component suitable for being received by a USMC connector of a prosthetic arm may be secured or fastened onto the thread of the mating part 22. Alternatively, any other component, such as a component suitable for another type of connector may be secured or fastened onto the thread of the mating part 22.

As shown in Figures 1 and 2, the mating part 22 may protrude from an external surface 24 of the wrist attachment part 20. Alternatively, the mating part 22 may be recessed into the wrist attachment part 20, such as into the external surface 24 of the wrist attachment part 20. The external surface 24 of the wrist attachment part 20 may be substantially circular. The external surface 24 may comprise a width, length, and/or diameter that is approximately or substantially equal to a width, length, and/or diameter of an end surface of a connecting prosthetic arm. Alternatively, the external surface 24 may comprise a width, length, and/or diameter that is approximately or substantially equal to a width, length, and/or diameter of an intermediate connecting plate. The intermediate connecting plate may be an interface that that connects or couples the wrist attachment part 20 to a prosthetic arm. The intermediate plate may be a wrist connector. Accordingly, the wrist attachment part 20 may be configured for connecting to a wrist connector. It is noted that the external surface 24 may alternatively comprise any other suitable shape, and may be made to any suitable dimension. For example, the external surface 24 may be oval, square, rectangular, triangular, etc. The external surface 24 may be of a shape that is substantially similar or approximate to a shape of an end surface of a connecting prosthetic arm. In some configurations, the end surface of the prosthetic arm or connecting plate may be configured to abut and/or be flush against the external surface 24. It is further noted that in some configurations, the prosthetic hand 10 may be rotatably fixed relative to the intermediate connecting plate and/or the connecting prosthetic arm. However, in other configurations, the prosthetic hand 10 may be free to rotate about a longitudinal axis of the forearm, i.e. in supination and pronation directions.

Referring still to Figures 1 and 2, the wrist attachment part 20 may comprise a casing 26. The external surface 24 may be formed at an end of the casing 26. In particular, the external surface 24 may be formed at an end of the casing 26 configured to be proximate to a connecting prosthetic arm. The casing 26 may comprise a first casing part 27 and a second casing part 28. The first casing part 27 and the second casing part 28 may be distinct parts. Alternatively, the first casing part 27 and the second casing part 28 may be unitary. The first casing part 27 and the second casing part 28 may be rigidly connected or coupled to one another. The first casing part 27 and the second casing part 28 may be fixedly connected or coupled to one another. Any suitable fixing, fastening, or securing means may be used to couple or connect the first casing part 27 and the second casing part 28 to one another. The first casing part 27 and the second casing part 28 may be detachable, decouplable, or otherwise separable from one another. For example, one of the first casing part 27 or the second casing part 28 may comprise at least one hole suitable to insert a screw, and one of the first casing part 27 or the second casing part 28 may comprise at least one corresponding hole suitable to receive the screw. Additionally or alternatively, at least one standoff nut may be disposed between the first casing part 27 and the second casing part 28. Accordingly, a first screw may be inserted through the first casing part 27 and received by one side of a corresponding standoff nut, and a second screw may be inserted through the second casing part 28 and received by the other side of the corresponding standoff nut. It is noted that these are but examples of coupling the first casing part 27 and the second casing part 28 to one another, and that the first casing part 27 may be connected or coupled to the second casing part 28 in a plurality of other alternative ways. It is further noted that providing a first casing part 27 that is distinct from a second casing part 28 may allow the wrist attachment part 20 to be quickly and easily disassembled. The casing 26 is not limited to comprising two distinct parts. Indeed, the casing 26 may comprise more than two distinct parts.

The palm part 30 is rotatably coupled to the wrist attachment part 20 to at least partially form a wrist joint 50 with an axis of rotation 51. The axis of rotation 51 may be a lateral axis of rotation. The lateral axis of rotation may be substantially perpendicular to a longitudinal axis of a forearm. As will be described in more detail below, the palm part 30 may rotate relative to the wrist attachment part 20 about the lateral axis. Accordingly, relative rotation of the palm part 30 and wrist attachment part 20 may permit extension and flexion of the prosthetic hand 10, e.g. relative to a user's forearm or prosthetic forearm.

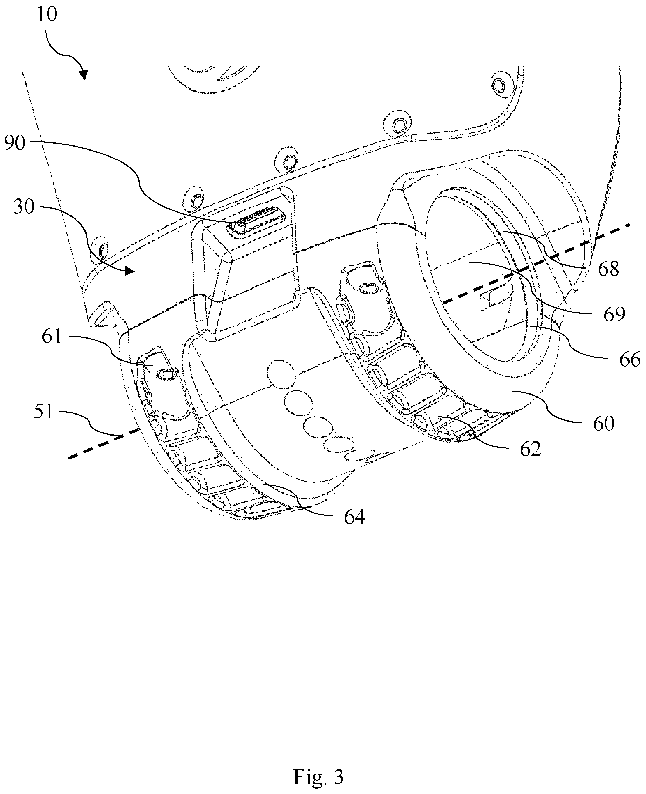

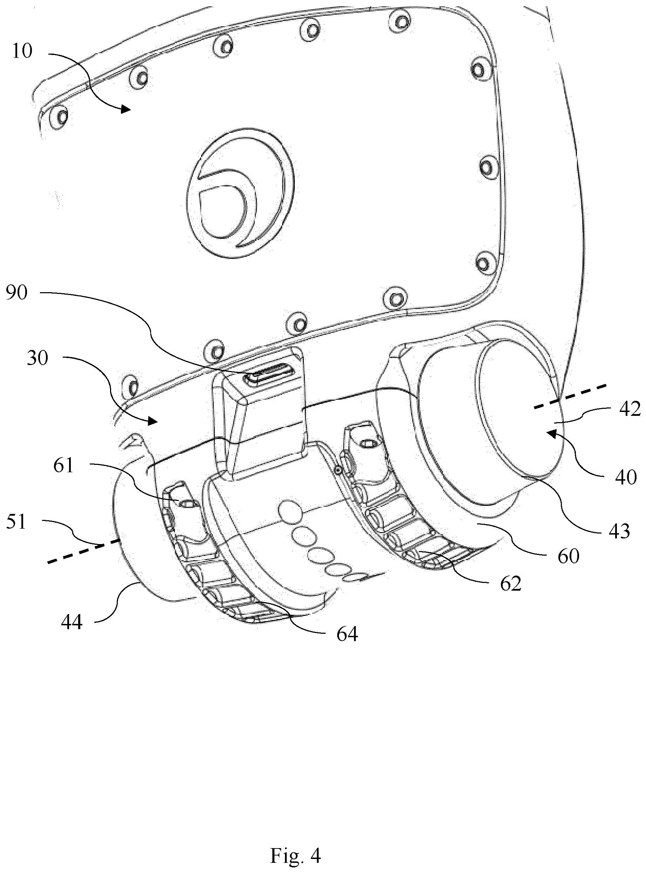

As depicted in Figures 3 and 4, the palm part 30 may comprise a bridging part 60. The bridging part 60 may be a distinct part to the palm part 30. In other words, the bridging part 60 may be considered to be a part in and of itself. The bridging part 60 may be couplable or connectable to the palm part 30. The bridging part 60 may be coupled or connected to the palm part 30 in such a manner as to be detachable, decouplable, and/or otherwise separable from the palm part 30. In other words, the bridging part 60 and the palm part 30 may be detachable, decouplable, and/or otherwise separable from one another. This may improve a modularization of the prosthetic hand 10. The bridging part 60 may be fixedly and/or rigidly connected to the palm part 30. For example, at least one screw, e.g. screw 61, may couple the bridging part 60 to the palm part 30. Alternatively, at least one screw may couple the palm part 30 to the bridging part 60. It is noted that the bridging part 60 and the palm part 30 may be coupled to one another using other alternative means. It is further noted that the bridging part 60 and the palm part 30 may be unitary. In other words, the bridging part 60 and the palm part 30 may be one part.

The bridging part 60 of the palm part 30 may be disposed or positioned proximate to the wrist attachment part 20. Specifically, the bridging part 60 of the palm part 30 may be at least partially surrounded or at least partially housed by the wrist attachment part 20. In particular, the bridging part 60 of the palm part 30 may be at least partially surrounded or at least partially housed by the casing 26 of the wrist attachment part 20. For example, the bridging part 60 of the palm part 30 may be at least partially surrounded or at least partially housed by the first casing part 27 and/or the second casing part 28 of the wrist attachment part 20.

As shown in Figures 2 and 4, a shaft 42 may extend through the palm part 30 and the wrist attachment part 20. The shaft 42 may therefore couple, or rotatable couple, the palm part 30 to the wrist attachment part 20. The shaft 42 may hinge the palm part 30 to the wrist attachment part 20. The shaft 42 may permit the palm part 30 to be pivotable relative to the wrist attachment part 20. The shaft 42 may comprise the battery housing 40. In other words, the shaft 42 may be the battery housing 40. Alternatively, the shaft 42 may comprise a cavity, and the cavity may house the battery housing 40. The shaft 42 may extend in a lateral direction of the wrist attachment part 20 and/or the palm part 30. The shaft 42 may extend in a lateral direction of the wrist attachment part 20 and/or the palm part 30 at least partially through the palm part 30 and/or at least partially through the wrist attachment part 20. For example, the shaft 42 may extend from the first casing part 27 of the wrist attachment part 20, into the bridging part 60 of the palm part 30, and into the second casing part 28 of the wrist attachment part 20. As shown in Figures 1 and 2, the wrist attachment part 20 may comprise at least one lateral side opening, e.g. lateral side opening 29. The at least one lateral side opening may be configured to permit the shaft 42 to be slotted or disposed into the wrist attachment part 20. Although only the lateral side opening 29 of the second casing part 28 is shown, e.g. as in Figures 1 and 2, it is envisaged that the first casing part 27 may comprise a corresponding lateral side opening.

Referring now to Figures 2 and 3, the shaft 42 may be slotted into one of the lateral side openings of the wrist attachment part 20, e.g. into either the lateral side opening 29 of the second casing part 28 or the corresponding lateral side opening of the first casing part 27. The shaft 42 may extend into the palm part 30. Specifically, the shaft 42 may extend through a cavity or through-hole 69 (see Figure 3) formed in the palm part 30. The cavity or through-hole 69 of the palm part 30 may be formed in the bridging part 60 of the palm part 30. The shaft 42 may therefore extend out of the cavity or through-hole 69 of the palm part 30 and into the other lateral side opening of the wrist attachment part 20, e.g. into one of lateral side opening 29 of the second casing part 28 or the corresponding lateral side opening of the first casing part 27. Accordingly, the shaft 42 may function to hinge and/or pivot the palm part 30 to the wrist attachment part 20. In other words, the shaft 42 may rotatably couple the palm part 30 to the wrist attachment part 20 to form a wrist joint 50 with an axis of rotation 51. A user may be able to flex and/or extend the palm part 30 about the axis of rotation 51.

Referring now to Figures 3 and 4, the palm part 30 may comprise at least notch, e.g. notch 62. Specifically, the bridging part 60 of the palm part 30 may comprise at least one notch 62. For example, a plurality of notches may be arranged on and/or around a surface of the bridging part 60 of the palm part 30. The notches 62 may be arranged on a surface of the bridging part 60 to permit the palm part 30 to be sequentially rotated, e.g. from one notch to another, about the axis of rotation 51. Although not shown, the wrist attachment part 20 may comprise at least one protrusion. In the absence of e.g. a compressive force, the protrusion of the wrist attachment part 20 may be configured to be in an extended position. When in the extended position, the protrusion may be configured to extend into or be received by a notch 62. This may lock rotation of the palm part 30 relative to the wrist attachment part 20. In other words, when a protrusion of the wrist attachment part 20 is received by a notch 62 of the bridging part 60, the palm part 30 may not be able to rotate relative to the wrist attachment part 20. The protrusion of the wrist attachment part 20 may be forced or otherwise urged, e.g. by a compressive force, into a retracted position. When in the retracted position, the corresponding protrusion may not extend into a notch 62. In this configuration, the palm part 30 may be permitted to rotate relative to the wrist attachment part 20. Specifically, the palm part 30 may be rotatable relative to the wrist attachment part 20 about axis of rotation 51. The at least one protrusion may be a resiliently flexible member. Accordingly, the at least one protrusion may snap in or out of a notch 62. In particular, the at least one protrusion may snap in or out of a notch 62 due to the palm part 30 being rotated relative to the wrist attachment part 20. The palm part 30 may therefore be rotatable through a series of discrete positions. For example, at a first locking position, a protrusion of the wrist attachment part 20 may be extended into or received by a notch 62 of the bridging part 60. A force may then be exerted on the palm part 30 or the wrist attachment part 20, such as to cause the protrusion of the wrist attachment part 20 to retract from the notch 62. The palm part 30 may then be rotated relative to the wrist attachment part 20. The palm part 30 may be rotated relative to the wrist attachment part 20 until the protrusion extends or snaps into a further notch 62. The palm part 30 may thus be locked into a second position. It is noted that the palm part 30, e.g. the bridging part 60 of the palm part 30, may instead comprise the at least one protrusion and the wrist attachment part 20 may comprise the at least one notch 62.

It is noted that this is but one example of an assembly or configuration in which the palm part 30 and wrist attachment part 20 are rotatably coupled together to at least partially form a wrist joint 50 with a lateral axis of rotation 51. The present disclosure is not limited to such an assembly or configuration. Indeed, the palm part 30 and wrist attachment part 20 may be rotatably coupled together to form a wrist joint 50 with an axis of rotation 51 by way of an alternative assembly or configuration. For example, at least one shaft or tab may extend from one of the wrist attachment part 20 or the palm part 30 into a corresponding cavity, through-hole, or recess formed in the other of the palm part 30 or the wrist attachment part 20. This may accordingly permit the palm part 30 to be rotatable relative to the wrist attachment part 20. Additionally or alternatively, the palm part 30 may comprise an additional or alternative coupling mechanism that permits the palm part 30 to be rotatable relative to the wrist attachment part 20 through a plurality of discrete positions. For example, the palm part 30 and/or the wrist attachment part 20 may comprise an additional or alternative coupling mechanism instead of, or as well as, the at least one notch 62 and the at least one protrusion. For example, the prosthetic hand 10 may comprise an infinite adjustment system. That is, the palm part 30 may be rotatable (e.g. extended or flexed) to any position relative to the wrist attachment part 20. This may be achievable through a suitable fit (e.g. a push fit, interference fit, or press fit) between a portion of the wrist attachment part 20 and a portion of the palm part 30.

Referring now to Figure 3, the bridging part 60 of the palm part 30 may comprise an external surface 64 and an internal surface 66. The at least one notch 62 may be formed on the external surface 64 of the bridging part 60. The bridging part 60 may be substantially curved. For example, at least a portion of the bridging part 60 may be substantially semi-circular. It is noted that the internal surface 66 may be of a different shape, e.g. of a different profile, to the external surface 64 of the bridging part 60. For example, at least a portion of the external surface 64 of the bridging part 60 may comprise a first profile (e.g. flat, curved, undulating, etc.), whilst at least a portion of the internal surface 66 of the bridging part 60 may comprise a second profile (e.g. flat, curved, undulating, etc.). As best shown in Figure 3, at least a portion of the internal surface 66 of the bridging part 60 may be substantially semicircular.

Still referring to Figure 3, an end of the palm part 30 proximate to the bridging part 60 may comprise a recess 68. The recess 68 may comprise a shape or profile that corresponds to the shape or profile of the bridging part 60. For example, at least a portion of the recess 68 of the palm part 30 may be substantially semicircular. Accordingly, the cavity or through-hole 69 through which the shaft 42 or battery housing 40 is configured to extend through may be formed when the bridging part 60 is coupled or connected to the palm part 30. The cavity or through-hole 69 may be formed by the space between the internal surface 66 of the bridging part 60 and the recess 68 of the palm part 30. In particular, and as best shown in Figure 3, where the internal surface 66 of the palm part 30 is semicircular, and the recess of the palm part 30 comprises a corresponding semicircular shape, then the cavity or through-hole 69 formed therein may be substantially circular. It is noted that a cross-sectional shape or area of the cavity or through-hole 69 may vary. For example, a cross-sectional shape or area of the cavity or through-hole 69 may vary along a longitudinal axis of the cavity or through-hole 69. The cavity or through-hole 69 formed may at least partially surround the battery housing 40. Accordingly, the cavity or through-hole 69 may be formed with any shape or shapes suitable for containing the battery housing 40. It is noted that the cavity or through-hole 69 may be formed by various methods. For example, the cavity or through-hole 69 may be formed in manufacture, e.g. in any suitable additive or subtractive manufacturing process. The cavity or through-hole 69 may extend at least partially through the palm part 30. In particular, the cavity or through-hole 69 may extend at least partially through the bridging part 60 of the palm part 30. The cavity or through-hole 69 may extend in a transverse or lateral direction of the palm part 30 and/or wrist attachment part 20 through the palm part 30. At least one end of the cavity or through-hole 69 may be aligned with at least one of the lateral side openings of the wrist attachment part 20. For example, one end of the cavity or through-hole 69 may be aligned with the lateral side opening 29 formed in the second casing part 28, and/or the other end of the cavity or through-hole 69 may be aligned with the corresponding lateral side opening formed in the first casing part 27. Accordingly, the shaft 42 or battery housing 40 may be slotted or disposed through the wrist attachment part 20 and the palm part 30, to thereby rotatably couple the two parts to one another.

Although the Figures show the cavity or through-hole 69 as a through-hole extending through the bridging part 60 of the palm part, it is noted that the present disclosure is not so limited. For example, in addition or as an alternative to the depicted cavity or through-hole 69, one of the palm part 30 (e.g. the bridging part 60 of the palm part 30) or the wrist attachment part 20 may comprise at least one cavity or recess, and the other of the palm part 30 (e.g. the bridging part 60 of the palm part 30) or the wrist attachment part 20 may comprise at least one corresponding tab or shaft. The at least one tab or shaft may be configured to extend into the at least one cavity or recess to thereby form a wrist joint 50 with an axis of rotation 51. The palm part 30 (e.g. the bridging part 60 of the palm part 30) may comprise a further cavity or a further recess, the battery housing may be disposed within the further cavity or further recess, such that the axis of rotation 51 of the wrist joint 50 passes through the battery housing 40.

As shown in Figures 1 and 2, the battery housing 40 is positioned such that the axis of rotation 51 of the wrist joint 50 passes through the battery housing 40. Referring to Figure 4, the battery housing 40 may be disposed in the palm part 30. The battery housing 40 may extend through the palm part 30 and the wrist attachment part 20. Specifically, the battery housing 40 may be disposed in at least the cavity or through-hole 69 of the bridging part 60. A longitudinal axis of the battery housing 40 may be substantially or approximately parallel, coincidental, and/or intersecting to the axis of rotation 51 of the wrist joint 50. A longitudinal axis of the battery housing 40 may be substantially or approximately superimposed over the axis of rotation 51 of the wrist joint 50. The cavity or through-hole 69 may be disposed or positioned through the palm part 30 such that, when the battery housing 40 is disposed in the cavity or through-hole 69, a longitudinal axis of the battery housing 40 may be substantially or approximately parallel, coincidental, intersecting and/or superimposed to the axis of rotation 51 of the wrist joint 50. As best shown in Figure 4, the battery housing 40 may comprise a first end 43 and a second end 44. The first end 43 and/or the second end 44 of the battery housing 40 may be configured to protrude or extend out of the cavity or through-hole 69 of the bridging part 60 of the palm part 30. Accordingly, at least one of the lateral side openings of the wrist attachment part 20 may be coupled, connected, or otherwise disposed around one of the first end 43 or the second end 44 of the battery housing 40. For example, lateral side opening 29 of the second casing part 28 may be disposed around the second end 44 of the battery housing 40, and/or the other lateral side opening of the first casing part 27 may be disposed around the first end 43 of the battery housing 40. As shown in Figures 1 and 2, the first end 43 and/or the second end 44 of the battery housing 40 may be flush against a corresponding lateral side opening of the wrist attachment part 20. Accordingly, the battery housing 40 may function as the shaft 42 that hinges the palm part 30 to the wrist attachment part 20. The battery housing 40 is therefore effectively used as both a shaft that permits the wrist attachment part 20 and the palm part 30 to rotate relative to one another, and as a housing for a battery. By co-locating these functions, a battery may be disposed in the battery housing 40 of the prosthetic hand 10 without limiting a range of motion of the palm part 30 of the prosthetic hand 10.

The battery housing 40 may be configured to be rotatable with the palm part 30 relative to the wrist attachment part 20. In other words, the battery housing 40 may be fixedly and/or rigidly coupled or connected to the palm part 30 and/or the bridging part 60, and rotatably coupled or connected to the wrist attachment part 20. Therefore, if the palm part 30 is rotated or pivoted relative to the wrist attachment part 20, e.g. when it is extended or flexed, the battery housing 40 may rotate with the palm part 30 relative to the wrist attachment part 20. Since the battery housing 40 may not rotate relative to the palm part 30, there may be a reduced likelihood of couplings (e.g. wires, cables, cords, etc.) that may couple a battery housed in the battery housing 40 to components (e.g. electrical components) comprised by the palm part 30, from becoming twisted, tangled, or otherwise worn, as the wires do not become stretched and/or compressed as the palm part 30 is extended and/or flexed. The battery housing 40 may be of any width and/or length. Accordingly, the battery housing 40 may be configured to house any type of battery cell, module, and/or pack. However, it is envisaged that the battery housing 40 may be configured to house an 18650 battery cell. The battery cell, module, and/or pack may be rechargeable. Recharging of a battery may occur in a plurality of different ways. For example, a battery housed in the battery housing 40 may be charged and/or recharged wirelessly, inductively, and/or otherwise 'cordlessly', e.g. by magnetic induction. Additionally or alternatively, the prosthetic hand 10 may comprise at least one port, such as port 90 shown in Figures 1 to 4. The port 90 may be configured to permit an external power source to be electrically coupled to a battery housed in the battery housing 40. This may permit a battery housed in the battery housing 40 to be charged and/or recharged. The port 90 may be a USB-C port. The port 90 may alternatively be a different type of port. Although not shown, there may be more than one port. Where there are a plurality of ports, each port may or may not be the same type of port. Although the port 90 is depicted as being disposed on the palm part 30, the port 90 may in fact be disposed anywhere on the prosthetic hand 10. For example, the port 90 may be disposed anywhere on the wrist attachment part 20 or the palm part 30.

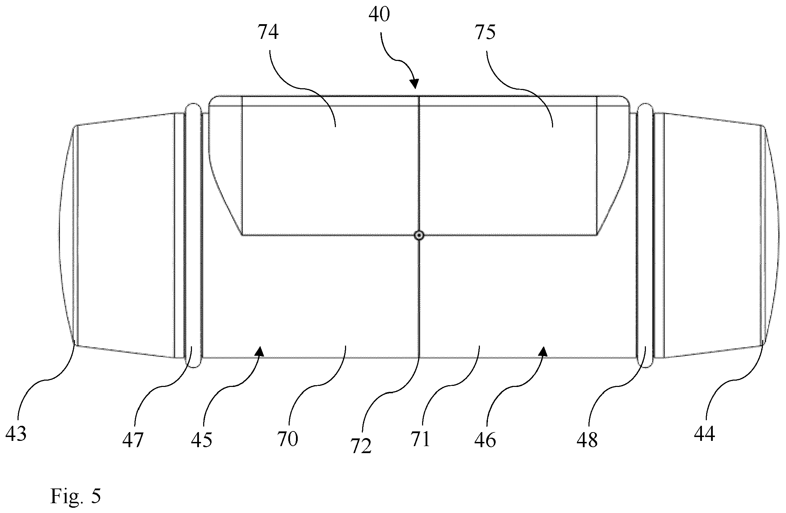

Referring now to Figure 5, the battery housing 40 may comprise at least one part. For example, the battery housing may comprise a first part 45 and a second part 46. The first part 45 and the second part 46 may be distinct parts. Alternatively, the first part 45 and the second part 46 may be unitary. The shape and/or dimensions of the first part 45 of the battery housing 40 may be substantially similar to the shape and/or dimensions of the second part 46 of the battery housing 40. Alternatively, the shape and/or dimensions of the first part 45 may at least partially differ to the shape and/or dimensions of the second part 46. The first part 45 and the second part 46 of the battery housing 40 may be rigidly and/or fixedly coupled or connected to one another. The first part 45 and the second part 46 of the battery housing may be decouplable or otherwise separable from one another. The first part 45 of the battery housing 40 may be configured to house a first portion, e.g. a first half, of a battery. The second part 46 of the battery housing 40 may be configured to house a second portion, e.g. the second half, of the battery. The first part 45 of the battery housing 40 may comprise first end 43. The second part 46 of the battery housing 40 may comprise second end 44. The first part 45 of the battery housing 40 may comprise a first joining portion 70. The second part 46 of the battery housing 40 may comprise a second joining portion 71. A leading surface of the first joining portion 70 may directly contact and/or abut a leading surface of the second joining portion 71. A leading surface of the first joining portion 70 may directly contact and/or abut a leading surface of the second joining portion 71 to define an interface surface 72. The interface surface 72 may be configured to at least partially surround a battery. The interface surface 72 may at least partially surround a geometric centre of the battery housing 40. It is noted that the first joining portion 70 and the second joining portion 71 may be unitary, i.e., the first joining portion 70 and the second joining portion 71 may be single portion that joins the first end 43 to the second end 44 of the battery housing.

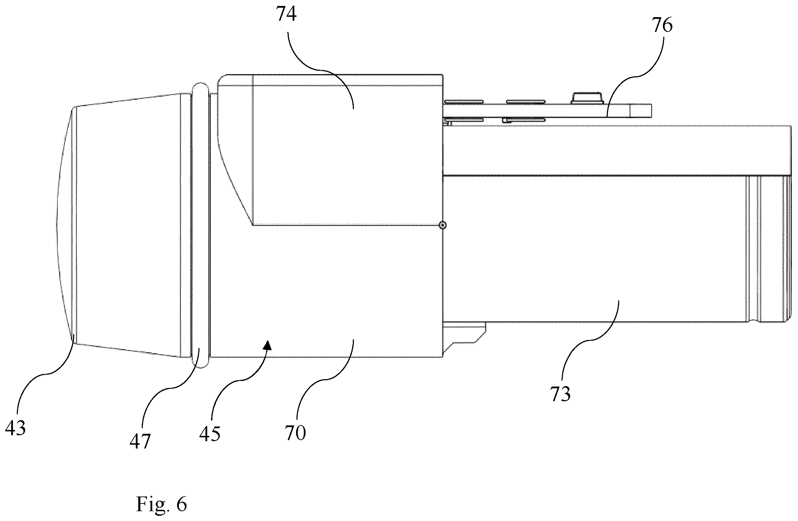

As shown in Figure 6, one or both of the first part 45 or the second part 46 of the battery housing 40 may be removed, replaced, or otherwise decoupled or disconnected from the battery housing 40. This may facilitate an ease of access to a battery, e.g. battery 73, contained within the battery housing 40. For example, an end-user, supplier, or manufacturer may ergonomically install, replace, or otherwise remove a battery from an assembled prosthetic hand by decoupling the first casing part 27 and/or the second casing part 28 of the wrist attachment part 20 from the prosthetic hand 10, decoupling the bridging part 60 from the prosthetic hand 10, and removing one or both of the first part 45 or the second part 46 of the battery housing 40. Additionally or alternatively, the first end 43 and/or the second end 44 of the battery housing 40 may be decouplable from the battery housing 40. In other words, the first end 43 and/or the second end 44 of the battery housing 40 may be removable caps or lids, e.g. a user may remove first end 43 and/or second end 44, remove a faulty battery disposed in the battery housing 40, insert a replacement battery, and re-install the first end 43 and/or the second end 44 of the battery housing 40.

As best shown in Figure 5, the first end 43 and/or the second end 44 of the battery housing 40 may comprise a truncated cone and/or be substantially frustoconical in shape. In other words, the first end 43 and/or the second end 44 of the battery housing 40 may comprise a smaller diameter surface and a larger diameter surface, and a tapered portion that joins the smaller diameter surface to the larger diameter surface. It is noted that lateral side opening 29 of the second casing part 28 of the wrist attachment part 20 may be disposed around a periphery of the smaller diameter surface of the second end 44, and/or the corresponding lateral side opening of the first casing part 27 of the wrist attachment part 20 may be disposed around a periphery of the smaller diameter surface of the first end 43. It is further noted that the first end 43 and/or the second end 44 of the battery housing 40 may be different in shape to one another, and/or comprise a shape that is not substantially frustoconical. For example, the first end 43 and/or the second end 44 of the battery housing 40 may be substantially cylindrical, cuboid, etc.

Referring to Figures 5 and 6, a cross-sectional shape and/or area of the first joining portion 70 and/or the second joining portion 71 may vary. In particular, a cross-sectional shape and/or area of the first joining portion 70 and/or the second joining portion 71 may vary along a longitudinal length of the battery housing 40. For example, towards or at the join or contact between the first joining portion 70 and the first end 43, the cross-sectional shape of the first joining portion 70 may be substantially similar to a cross-sectional shape of the first end 43. More specifically, towards or at the join or contact between the first joining portion 70 and the first end 43, the cross-sectional shape of the first joining portion 70 may be substantially similar to a cross-sectional shape of the larger diameter surface of the first end 43. Similarly, towards or at the join or contact between the second joining portion 71 and the second end 44, the cross-sectional shape of the second joining portion 71 may be substantially similar to a cross-sectional shape of the second end 44. More specifically, towards or at the join or contact between the second joining portion 71 and the second end 44, the cross-sectional shape of the second joining portion 71 may be substantially similar to a cross-sectional shape of the larger diameter surface of the second end 44.

Still referring to Figures 5 and 6, the battery housing 40 may comprise at least one enlargement part, such as first enlargement part 74 and/or second enlargement part 75. First enlargement part 74 may be coupled or connected to the first part 45 of the battery housing 40. Second enlargement part 75 may be coupled or connected to the second part 46 of the battery housing 40. First enlargement part 74 may be coupled or connected to second enlargement part 75. First enlargement part 74 and first part 45 of the battery housing 40 may be unitary. Second enlargement part 75 and second part 46 of the battery housing 40 may be unitary. As best shown in Figure 6, first enlargement part 74 and/or second enlargement part 75 may be configured to increase an internal volume of the battery housing 40. For example, first enlargement part 74 and/or second enlargement part 75 may increase a width, length, and/or height of the battery housing 40. First enlargement part 74 and/or second enlargement part 75 may be configured to increase an internal volume of the battery housing 40 to thereby permit other components, such as a protection board, circuit board 76 and/or any other electrical components, to also be housed in the battery housing 40. Circuit board 76 and/or the any other components may be configured housed in the battery housing 40 adjacent to the battery 73. Accordingly, the battery housing 40 may be configured to house a circuit board, and/or a protection board, and/or any other component alongside a battery. First enlargement part 74 and/or second enlargement part 75 may be disposed towards the palm part 30. First enlargement part 74 and/or second enlargement part 75 may be configured to overhang or extend outwards over a battery. For example, first enlargement part 74 and/or second enlargement part 75 may be configured to overhang or extend outwards over battery 73 towards the palm part 30. This may improve an ability to couple the battery to other electrical components of the prosthetic hand 10, as wiring or cables configured to couple battery 73 to other components, such as to a drive system in the prosthetic hand 10, may extend from the at least one enlargement part into the prosthetic hand 10, e.g. from circuit board 76 into the palm part 30 of the prosthetic hand 10. This may also improve an ability to shield or protect a battery from substances that may potentially damage or otherwise impair a battery, such as by reducing a likelihood of moisture or water from entering the battery housing 40.

As shown in Figures 5 and 6, the battery housing 40 may comprise at least one sealing ring. For example, a first sealing ring 47 may be disposed towards the first end 43 of the battery housing 40, and/or a second sealing ring 48 may be disposed towards the second end 44 of the battery housing 40. The battery housing 40 may comprise at least one groove configured to locate the at least one sealing ring. The first sealing ring 47 may be disposed between the first enlargement part 74 and the first end 43 of the battery housing 40. The second sealing ring 48 may be disposed between the second enlargement part 75 and the second end 44 of the battery housing 40. The first and/or the second sealing ring may therefore reduce a likelihood of a substance or a fluid from interfering with cables or wiring extending from a battery housed in the battery housing 40. It is noted that the first sealing ring 47 and/or the second sealing ring 48 may be disposed elsewhere on the battery housing 40, and that the battery housing may comprise more than two sealing rings. The battery housing 40 may comprise any number of sealing rings. The sealing rings may be O-rings, gaskets, and/or any other type of sealing ring. In addition to or as an alternative to the at least one sealing ring, the battery housing 40 may comprise a different kind of sealant or sealing configuration. For example, the battery housing 40 may comprise at least one layer of sealant, such as at least one layer of silicone or polyurethane sealant.

As shown in Figure 2, the prosthetic hand 10 may comprise at least one digit. In particular, the palm part 30 may be couplable to at least one prosthetic digit. For example, the palm part may be coupled to a first digit 80, a second digit 81, a third digit 82, a fourth digit 83, and a fifth digit 84. The first digit 80 may be a prosthetic thumb, the second digit 81 may be a prosthetic index finger, the third digit 82 may be a prosthetic middle finger, the fourth digit 83 may be a prosthetic ring finger, and the fifth digit 84 may be a prosthetic little finger. It is noted that the prosthetic hand 10 may comprise any number of prosthetic digits. It is further noted that the prosthetic digits may be of any shape or size. **In** other words, the prosthetic digits are not limited to being of the shapes and/or sizes depicted in Figure 2.

Although not shown, the prosthetic hand 10 may comprise at least one driving system configured to control a movement of the at least one prosthetic digit. The at least one driving system may be disposed in the palm part 30 of the prosthetic hand 10. There may be one driving system per digit. The at least one driving system may comprise any suitable combination of actuators, shafts, gears, etc. (or any other additional or alternative mechanisms) that facilitate control of the at least one prosthetic digit. The at least one driving system, and/or any other electrical components that may be present in the prosthetic hand 10, may be configured to communicate (e.g. via at least one electrical coupling, such as at least one wire or cable) with a battery housed in the battery housing 40. In other words, the at least one driving system, and/or any other electrical components that may be present in the prosthetic hand 10, may be powered by a battery, e.g. battery 73, housed in the battery housing 40.

The at least one driving system, battery, or batteries, and/or any other electrical components of the prosthetic hand 10 may be controlled by a controller. The controller may be disposed within the prosthetic hand 10. The controller may be disposed elsewhere on a user, such as elsewhere on a user's prosthesis, e.g. on the prosthetic arm of a user. The controller may be remote to the prosthetic hand 10. For example, the controller may be implemented on any suitable device, such as a laptop, desktop, mobile device, etc. There may be more than one controller. Where there are a plurality of controllers, the controllers may or may not be disposed proximate to one another. For example, a first controller may be disposed on a user's prosthetic arm. A second controller may be disposed adjacent to the first controller or may alternatively be disposed elsewhere, such as in or on the prosthetic hand 10. The controller or controllers may be configured to control electrical components within the prosthetic hand 10, such as the at least one driving system, based on at least one received signal. The at least one received signal may be generated by at least one sensor. The at least one sensor may comprise at least one electromyography (EMG) sensor. The at least one sensor may however be a different type of sensor. The at least one sensor may comprise a plurality of different types of sensors. The at least one sensor may be configured to be disposed on or adjacent to a user's residual limb. The residual limb may be a residual arm of a user. The at least one sensor and the at least one controller may be configured to communicate wirelessly. Similarly, the at least one controller and electrical components within the prosthetic hand 10 may be configured to communicate wirelessly. The at least one sensor and electrical components within the prosthetic hand 10 may be configured to communicate wirelessly. It is envisaged that wireless communication may be via Bluetooth^{®}. However, wireless communication may additionally or alternatively be via any other wireless communication protocol. Therefore, since electrical components of the prosthetic hand 10 may be powered by a battery (e.g. battery 73) disposed locally in the battery housing 40 of the prosthetic hand 10, and communication for controlling components of the prosthetic hand 10 may occur wirelessly, a number and/or length of wiring and/or cables required for a prosthetic hand 10 and/or a prosthetic system (e.g. a prosthetic hand 10 connected to a prosthetic arm) may be substantially reduced or eliminated.

Although the above description focusses on a prosthetic hand, it is also envisaged that the present invention may be applied to other terminal devices, such as a prosthetic foot. For example, a foot part and an ankle attachment part may be rotatably coupled together to at least partially form an ankle joint with a lateral axis of rotation. A battery housing may be positioned such that the axis of rotation of the ankle joint passes through the battery housing. The ankle attachment part may be configured for connecting to a prosthetic leg and/or an intermediate ankle connecting plate. The ankle attachment part may be provided at a distal end of a prosthetic leg or lower leg.

Variations to the disclosed embodiments can be understood and effected by those skilled in the art in practicing the principles and techniques described herein, from a study of the drawings, the disclosure and the appended claims. **In** the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A prosthetic hand comprising:
a wrist attachment part configured for connecting to a prosthetic arm;
a palm part, **characterized by** the palm part and wrist attachment part being rotatably coupled together to at least partially form a wrist joint with a lateral axis of rotation; and
a battery housing, wherein the battery housing is positioned such that the axis of rotation of the wrist joint passes through the battery housing.

2. The prosthetic hand of claim 1, wherein the battery housing forms a shaft configured to rotatably couple the wrist attachment portion and the palm part.

3. The prosthetic hand of claim 1, wherein a longitudinal axis of the battery housing is substantially parallel and coincidental to the lateral axis of rotation of the wrist joint.

4. The prosthetic hand of any preceding claim, wherein a cavity at least partially extends through the palm part, and wherein the battery housing is disposed in the cavity.

5. The prosthetic hand of any preceding claim, wherein the wrist attachment part comprises a first casing part and a second casing part, wherein the first casing part at least partially surrounds a first portion of the palm part, and the second casing part at least partially surrounds a second portion of the palm part, and wherein the first casing part and the second casing part of the wrist attachment part are decouplable from one another.

6. The prosthetic hand of any of claims 1 to 5, wherein the wrist attachment part comprises a first lateral side opening and a second lateral side opening, and the palm part comprises a through hole, the first lateral side opening of the wrist attachment part being aligned to one end of the through-hole and the second lateral side opening of the wrist attachment part being aligned to the other end of the through-hole, and wherein the battery housing extends from the first lateral side opening of the wrist attachment part, through the through-hole of the palm part, and into the second lateral side opening of the wrist attachment part, to thereby rotatably couple the palm part to the wrist attachment part.

7. The prosthetic hand of claim 6, wherein the palm part is coupled to a bridging part, the bridging part being disposed proximate to the wrist attachment part and being at least partially surrounded by the wrist attachment part, and wherein the through-hole is formed between the bridging part and the palm part, and optionally wherein the bridging part and the palm part are decouplable from one another.

8. The prosthetic hand of claim 7, wherein the wrist attachment part comprises a first casing part and a second casing part, and wherein the first casing part at least partially surrounds a first portion of the bridging part, and the second casing part at least partially surrounds a second portion of the bridging part, and optionally wherein:
the first casing part and the second casing part of the wrist attachment part are decouplable from one another; or
the first casing part comprises the first lateral side opening, and the second casing part comprises the second lateral side opening.

9. The prosthetic hand of any of claims 6 to 8, wherein:
the battery housing comprises a first frustoconical end and a second frustoconical end, and
the first lateral side opening of the wrist attachment part is disposed around a periphery of a smaller diameter surface of the first frustoconical end, and the second lateral side opening of the wrist attachment part is disposed around a periphery of a smaller diameter surface of the second frustoconical end; and optionally wherein:
the battery housing comprises a first part and a second part, the first part of the battery housing being separable from the second part of the battery housing, the first part of the battery housing comprising the first frustoconical end and a joining portion, the second part of the battery housing comprising the second frustoconical end and a joining portion, and
a leading surface of the joining portion of the first part of the battery housing abuts a leading surface of the joining portion of the second part of the battery housing to define an interface surface, the interface surface being configured to surround a battery.

10. The prosthetic hand of any preceding claim, wherein the battery housing comprises a first part and a second part, the first part being separable from the second part, the first part being configured to house a first portion of a battery and the second part being configured to house a second portion of the battery.

11. The prosthetic hand of any preceding claim, wherein the palm part is couplable to at least one prosthetic digit, wherein the prosthetic hand comprises at least one driving system configured to control a movement of the at least one prosthetic digit, and wherein the at least one driving system is configured to be electrically coupled to a battery housed in the battery housing, and optionally wherein at least one controller is configured to control the at least one driving system based on at least one received signal, and wherein the at least one received signal is configured to be wirelessly communicated to the controller.

12. The prosthetic hand of any preceding claim, wherein the battery housing is configured to house a rechargeable battery, and wherein one of the palm part or the wrist attachment part comprises a port, the port being configured to permit an external power source to be electrically coupled to the rechargeable battery.

13. The prosthetic hand of any preceding claim, wherein the battery housing comprises at least one enlargement part, the enlargement part being configured to increase an internal volume of the battery housing.

14. The prosthetic hand of any preceding claim, wherein an external surface of the battery housing comprises at least one groove, the at least one groove being configured to locate a sealing ring.

15. The prosthetic hand of claim 13, wherein the battery housing comprises a first groove and a second groove, each groove being configured to locate a sealing ring, and wherein the first groove is disposed between the at least one enlargement part and a first end of the battery housing, and the second groove is disposed between the at least one enlargement part and a second end of the battery housing.

## Patentansprüche

1. Prothetische Hand, umfassend:
ein Handgelenkbefestigungsteil, das zum Verbinden mit einem prothetischen Arm ausgebildet ist;
ein Handflächenteil, **gekennzeichnet durch**
das drehbare Koppeln des Handflächenteils und des Handgelenkbefestigungsteils miteinander, um zumindest teilweise ein Handgelenk mit einer seitlichen Drehachse zu bilden; und
ein Batteriegehäuse, wobei das Batteriegehäuse derart positioniert ist, dass die Drehachse des Handgelenks durch das Batteriegehäuse verläuft.

2. Prothetische Hand nach Anspruch 1, wobei das Batteriegehäuse eine Welle bildet, die dazu ausgebildet ist, den Handgelenkbefestigungsabschnitt und das Handflächenteil drehbar zu koppeln.

3. Prothetische Hand nach Anspruch 1, wobei eine Längsachse des Batteriegehäuses im Wesentlichen parallel und deckungsgleich zu der seitlichen Drehachse des Handgelenks ist.

4. Prothetische Hand nach einem vorhergehenden Anspruch, wobei sich ein Hohlraum zumindest teilweise durch das Handflächenteil erstreckt und wobei das Batteriegehäuse in dem Hohlraum angeordnet ist.

5. Prothetische Hand nach einem vorhergehenden Anspruch, wobei das Handgelenkbefestigungsteil ein erstes Gehäuseteil und ein zweites Gehäuseteil umfasst, wobei das erste Gehäuseteil einen ersten Abschnitt des Handflächenteils zumindest teilweise umgibt und das zweite Gehäuseteil einen zweiten Abschnitt des Handflächenteils zumindest teilweise umgibt, und wobei das erste Gehäuseteil und das zweite Gehäuseteil des Handgelenkbefestigungsteils voneinander entkoppelbar sind.

6. Prothetische Hand nach einem der Ansprüche 1-5, wobei das Handgelenkbefestigungsteil eine erste seitliche Öffnung und eine zweite seitliche Öffnung umfasst und das Handflächenteil ein Durchgangsloch umfasst, wobei die erste seitliche Öffnung des Handgelenkbefestigungsteils auf ein Ende des Durchgangslochs ausgerichtet ist und die zweite seitliche Öffnung des Handgelenkbefestigungsteils auf das andere Ende des Durchgangslochs ausgerichtet ist, und wobei sich das Batteriegehäuse von der ersten seitlichen Öffnung des Handgelenkbefestigungsteils durch das Durchgangsloch des Handflächenteils hindurch und in die zweite seitliche Öffnung des Handgelenkbefestigungsteils erstreckt, um dadurch das Handflächenteil drehbar mit dem Handgelenkbefestigungsteil zu koppeln.

7. Prothetische Hand nach Anspruch 6, wobei das Handflächenteil mit einem Brückenteil gekoppelt ist, wobei das Brückenteil nahe dem Handgelenkbefestigungsteil angeordnet und zumindest teilweise von dem Handgelenkbefestigungsteil umgeben ist, und wobei das Durchgangsloch zwischen dem Brückenteil und dem Handflächenteil ausgebildet ist, und wobei optional das Brückenteil und das Handflächenteil voneinander entkoppelbar sind.

8. Prothetische Hand nach Anspruch 7, wobei das Handgelenkbefestigungsteil ein erstes Gehäuseteil und ein zweites Gehäuseteil umfasst, und wobei das erste Gehäuseteil einen ersten Abschnitt des Brückenteils zumindest teilweise umgibt und das zweite Gehäuseteil einen zweiten Abschnitt des Brückenteils zumindest teilweise umgibt, und wobei optional:
das erste Gehäuseteil und das zweite Gehäuseteil des Handgelenkbefestigungsteils voneinander entkoppelbar sind; oder
das erste Gehäuseteil die erste seitliche Öffnung umfasst und das zweite Gehäuseteil die zweite seitliche Öffnung umfasst.

9. Prothetische Hand nach einem der Ansprüche 6-8, wobei:
das Batteriegehäuse ein erstes kegelstumpfförmiges Ende und ein zweites kegelstumpfförmiges Ende umfasst und
die erste seitliche Öffnung des Handgelenkbefestigungsteils um einen Umfang einer Fläche mit kleinerem Durchmesser des ersten kegelstumpfförmigen Endes herum angeordnet ist und die zweite seitliche Öffnung des Handgelenkbefestigungsteils um einen Umfang einer Fläche mit kleinerem Durchmesser des zweiten kegelstumpfförmigen Endes herum angeordnet ist;
und optional wobei:
das Batteriegehäuse einen ersten Teil und einen zweiten Teil umfasst, wobei der erste Teil des Batteriegehäuses von dem zweiten Teil des Batteriegehäuses trennbar ist, wobei der erste Teil des Batteriegehäuses das erste kegelstumpfförmige Ende und einen Verbindungsabschnitt umfasst, wobei der zweite Teil des Batteriegehäuses das zweite kegelstumpfförmige Ende und einen Verbindungsabschnitt umfasst, und
eine Stirnfläche des Verbindungsabschnitts des ersten Teils des Batteriegehäuses an einer Stirnfläche des Verbindungsabschnitts des zweiten Teils des Batteriegehäuses anliegt, um eine Grenzfläche zu definieren, wobei die Grenzfläche dazu ausgebildet ist, eine Batterie zu umgeben.

10. Prothetische Hand nach einem vorhergehenden Anspruch, wobei das Batteriegehäuse einen ersten Teil und einen zweiten Teil umfasst, wobei der erste Teil von dem zweiten Teil trennbar ist, wobei der erste Teil dazu ausgebildet ist, einen ersten Abschnitt einer Batterie aufzunehmen, und der zweite Teil dazu ausgebildet ist, einen zweiten Abschnitt der Batterie aufzunehmen.

11. Prothetische Hand nach einem vorhergehenden Anspruch, wobei das Handflächenteil mit mindestens einem prothetischen Finger koppelbar ist, wobei die prothetische Hand mindestens ein Antriebssystem umfasst, das dazu ausgebildet ist, eine Bewegung des mindestens einen prothetischen Fingers zu steuern, und wobei das mindestens eine Antriebssystem dazu ausgebildet ist, elektrisch mit einer in dem Batteriegehäuse untergebrachten Batterie gekoppelt zu werden, und wobei optional mindestens eine Steuerung dazu ausgebildet ist, das mindestens eine Antriebssystem auf der Grundlage mindestens eines empfangenen Signals zu steuern, und wobei das mindestens eine empfangene Signal dazu ausgebildet ist, drahtlos an die Steuerung übermittelt zu werden.

12. Prothetische Hand nach einem vorhergehenden Anspruch, wobei das Batteriegehäuse dazu ausgebildet ist, eine wiederaufladbare Batterie aufzunehmen, und wobei eines von dem Handflächenteil oder dem Handgelenkbefestigungsteil einen Anschluss umfasst, wobei der Anschluss dazu ausgebildet ist, zu ermöglichen, dass eine externe Stromquelle elektrisch mit der wiederaufladbaren Batterie gekoppelt wird.

13. Prothetische Hand nach einem vorhergehenden Anspruch, wobei das Batteriegehäuse mindestens ein Erweiterungsteil umfasst, wobei das Erweiterungsteil dazu ausgebildet ist, ein Innenvolumen des Batteriegehäuses zu vergrößern.

14. Prothetische Hand nach einem vorhergehenden Anspruch, wobei eine Außenfläche des Batteriegehäuses mindestens eine Nut umfasst, wobei die mindestens eine Nut dazu ausgebildet ist, einen Dichtungsring aufzunehmen.

15. Prothetische Hand nach Anspruch 13, wobei das Batteriegehäuse eine erste Nut und eine zweite Nut umfasst, wobei jede Nut dazu ausgebildet ist, einen Dichtungsring aufzunehmen, und wobei die erste Nut zwischen dem mindestens einen Erweiterungsteil und einem ersten Ende des Batteriegehäuses angeordnet ist und die zweite Nut zwischen dem mindestens einen Erweiterungsteil und einem zweiten Ende des Batteriegehäuses angeordnet ist.

## Revendications

1. Prothèse de main comprenant :
une partie de fixation au poignet configurée pour se connecter à un bras prothétique ;
une partie de paume, **caractérisée par**
la partie de paume et la partie de fixation au poignet étant couplées ensemble de manière rotative pour former au moins partiellement une articulation du poignet avec un axe de rotation latéral ; et
un boîtier de batterie, dans laquelle le boîtier de batterie est positionné de sorte que l'axe de rotation de l'articulation du poignet passe à travers le boîtier de batterie.

2. Prothèse de main selon la revendication 1, dans laquelle le boîtier de batterie forme un arbre configuré pour coupler de manière rotative la partie de fixation au poignet et la partie de paume.

3. Prothèse de main selon la revendication 1, dans laquelle un axe longitudinal du boîtier de batterie est sensiblement parallèle et coïncident avec l'axe de rotation latéral de l'articulation du poignet.

4. Prothèse de main selon une quelconque revendication précédente, dans laquelle une cavité s'étend au moins partiellement à travers la partie de paume, et dans laquelle le boîtier de batterie est disposé dans la cavité.

5. Prothèse de main selon une quelconque revendication précédente, dans laquelle la partie de fixation au poignet comprend une première partie de carter et une seconde partie de carter, dans laquelle la première partie de carter entoure au moins partiellement une première partie de paume, et la seconde partie de carter entoure au moins partiellement une seconde partie de paume, et dans laquelle la première partie de carter et la seconde partie de carter de la partie de fixation au poignet sont découplables l'une de l'autre.

6. Prothèse de main selon l'une quelconque des revendications 1 à 5, dans laquelle la partie de fixation au poignet comprend une première ouverture latérale et une seconde ouverture latérale, et la partie de paume comprend un trou traversant, la première ouverture latérale de la partie de fixation au poignet étant alignée à une extrémité du trou traversant et la seconde ouverture latérale de la partie de fixation au poignet étant alignée à l'autre extrémité du trou traversant, et dans laquelle le boîtier de batterie s'étend à partir de la première ouverture latérale de la partie de fixation au poignet, à travers le trou traversant de la partie de paume, et dans la seconde ouverture latérale de la partie de fixation au poignet, pour ainsi coupler de manière rotative la partie de paume à la partie de fixation au poignet.

7. Prothèse de main selon la revendication 6, dans laquelle la partie de paume est couplée à une partie de pontage, la partie de pontage étant disposée à proximité de la partie de fixation au poignet et étant au moins partiellement entourée par la partie de fixation au poignet, et dans laquelle le trou traversant est formé entre la partie de pontage et la partie de paume, et éventuellement dans laquelle la partie de pontage et la partie de paume sont découplables l'une de l'autre.

8. Prothèse de main selon la revendication 7, dans laquelle la partie de fixation au poignet comprend une première partie de carter et une seconde partie de carter, et dans laquelle la première partie de carter entoure au moins partiellement une première portion de la partie de pontage, et la seconde partie de carter entoure au moins partiellement une seconde portion de la partie de pontage, et éventuellement dans laquelle :
la première partie de carter et la seconde partie de carter de la partie de fixation au poignet sont découplables l'une de l'autre ; ou
la première partie de carter comprend la première ouverture latérale, et la seconde partie de carter comprend la seconde ouverture latérale.

9. Prothèse de main selon l'une quelconque des revendications 6 à 8, dans laquelle :
le boîtier de batterie comprend une première extrémité tronconique et une seconde extrémité tronconique, et
la première ouverture latérale de la partie de fixation au poignet est disposée autour d'une périphérie d'une surface de plus petit diamètre de la première extrémité tronconique, et la seconde ouverture latérale de la partie de fixation au poignet est disposée autour d'une périphérie d'une surface de plus petit diamètre de la seconde extrémité tronconique ;
et éventuellement dans laquelle :
le boîtier de batterie comprend une première partie et une seconde partie, la première partie du boîtier de batterie pouvant être séparée de la seconde partie du boîtier de batterie, la première partie du boîtier de batterie comprenant la première extrémité tronconique et une portion de jonction, la seconde partie du boîtier de batterie comprenant la seconde extrémité tronconique et une portion de jonction, et
une surface d'attaque de la portion de jonction de la première partie du boîtier de batterie vient en butée contre une surface d'attaque de la portion de jonction de la seconde partie du boîtier de batterie pour définir une surface d'interface, la surface d'interface étant configurée pour entourer une batterie.

10. Prothèse de main selon une quelconque revendication précédente, dans laquelle le boîtier de batterie comprend une première partie et une seconde partie, la première partie pouvant être séparée de la seconde partie, la première partie étant configurée pour loger une première portion d'une batterie et la seconde partie étant configurée pour loger une seconde portion de la batterie.

11. Prothèse de main selon une quelconque revendication précédente, dans laquelle la partie de paume est couplable à au moins un doigt prothétique, dans laquelle la prothèse de main comprend au moins un système d'entraînement configuré pour commander un mouvement de l'au moins un doigt prothétique, et dans laquelle l'au moins un système d'entraînement est configuré pour être couplé électriquement à une batterie logée dans le boîtier de batterie, et éventuellement dans laquelle au moins un dispositif de commande est configuré pour commander l'au moins un système d'entraînement sur la base d'au moins un signal reçu, et dans laquelle l'au moins un signal est configuré pour être communiqué sans fil au dispositif de commande.

12. Prothèse de main selon une quelconque revendication précédente, dans laquelle le boîtier de batterie est configuré pour loger une batterie rechargeable, et dans laquelle l'une de la partie de paume ou de la partie de fixation au poignet comprend un port, le port étant configuré pour permettre à une source d'alimentation externe d'être couplée électriquement à la batterie rechargeable.

13. Prothèse de main selon une quelconque revendication précédente, dans laquelle le boîtier de batterie comprend au moins une partie d'élargissement, la partie d'élargissement étant configurée pour augmenter un volume interne du boîtier de batterie.

14. Prothèse de main selon une quelconque revendication précédente, dans laquelle une surface externe du boîtier de batterie comprend au moins une rainure, l'au moins une rainure étant configurée pour recevoir une bague d'étanchéité.

15. Prothèse de main selon la revendication 13, dans laquelle le boîtier de batterie comprend une première rainure et une seconde rainure, chaque rainure étant configurée pour recevoir une bague d'étanchéité, et dans laquelle la première rainure est disposée entre l'au moins une partie d'élargissement et une première extrémité du boîtier de batterie, et la seconde rainure est disposée entre l'au moins une partie d'élargissement et une seconde extrémité du boîtier de batterie.
